(19) Europäisches Patentamt
European Patent Office
Office européen des brevets

(11) Numéro de publication: **0 216 659**
**B1**

(12) **FASCICULE DE BREVET EUROPEEN**

(45) Date de publication du fascicule du brevet: **08.03.89**

(51) Int. Cl.⁴: **C07C 69/96**, C07C 68/02

(21) Numéro de dépôt: **86401804.9**

(22) Date de dépôt: **13.08.86**

(54) **Nouveaux halogénoformiates de dihalogéno-2,2 vinyle, leur procédé de préparation et leurs applications.**

(30) Priorité: **23.08.85 FR 8512652**

(43) Date de publication de la demande: **01.04.87 Bulletin 87/14**

(45) Mention de la délivrance du brevet: **08.03.89 Bulletin 89/10**

(84) Etats contractants désignés: **CH DE FR GB IT LI**

(56) Documents cités: **DE-A- 121 223**

(73) Titulaire: **SOCIETE NATIONALE DES POUDRES ET EXPLOSIFS, 12, quai Henri IV, F-75181 Paris Cédex 04(FR)**

(72) Inventeur: **Bowman, Mark Paul, 508 Fraser Street, State College Pennsylvania 16801(US)**
Inventeur: **Olofson, Roy Arne, 529 Cricklewood Drive, State College Pennsylvania 16803(US)**
Inventeur: **Malfroot, Thierry, 17, Chemin des Jardins, F-91100 Saintry-sur-Seine(FR)**
Inventeur: **Senet, Jean-Pierre, 79, rue de la Gare Herbeauvilliers Buthiers, F-77760 La Chapelle-la-Reine(FR)**

**EP 0 216 659 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

L'invention concerne de nouveaux halogénoformiates de vinyle dihalogénés en 2, leur procédé de préparation et leurs applications.

Quelques halogénoformiates insaturés sont connus et décrits dans la littérature. Ainsi le brevet US 2 377 085 concerne les chloroformiates de vinyle et de méthyl-2 vinyle et le brevet FR 2 421 866 le chloroformiate d'isopropényle.

Le fluoroformiate de vinyle a été préparé par P. Beak et J.A. Barron (J. Org. Chem. 1973, 38 (16) pp 2771–2775) et l'iodoformiate de vinyle par H.M.R. Hoffman et L. Iranshahi (J. Org. Chem. 1984, 49 pp 1174–1176).

Par contre on ne trouve aucune référence se rapportant à des halogénoformiates de dihalogénovinyle. Or ces composés sont très utiles pour introduire dans une molécule la fonction dihalogénovinyle.

Il est par conséquent d'un grand intérêt de pouvoir disposer de cette catégorie de composés et de leur procédé de préparation.

Les nouveaux halogénoformiates selon l'invention sont représentés par la formule

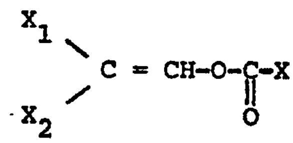

dans laquelle:
X signifie un atome de chlore ou de brome
$X_1$ et $X_2$ identiques ou différents signifient des atomes de chlore ou de brome.

En particulier l'invention concerne les composés pour lesquels X représente un atome de chlore et $X_1$ et $X_2$ sont identiques et signifient des atomes de chlore ou de brome, c'est-à-dire le chloroformiate de dichloro-2,2 vinyle

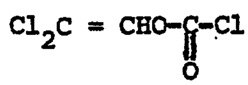

et le chloroformiate de dibromo-2,2 vinyle

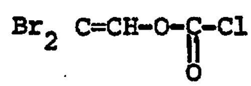

L'invention vise également le procédé de préparation des nouveaux halogénoformiates.

Ce procédé consiste à faire réagir un halogénoformiate de tétrahalogéno-1,2,2,2 éthyle de formule

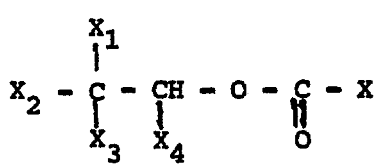

dans laquelle:
$X_1$, $X_2$ et X sont tels que définis précédemment, $X_3$ représente un atome de chlore ou de brome et représente toujours un atome de brome lorsque $X_1$ et/ou $X_2$ représente un atome de brome, $X_4$ représente un atome de chlore ou de brome,
avec un métal tel que le zinc ou le magnésium dans un milieu solvant.

Le schéma réactionnel est le suivant:

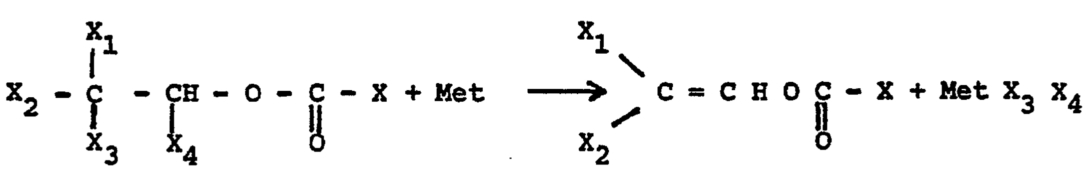

Il est surprenant d'obtenir par ce procédé des halogénoformiates insaturés dihalogénés car il était connu que les métaux comme le zinc ou les acides de Lewis, par exemple $ZnCl_2$, provoquaient une décomposition des chloroformiates par décarboxylation (M. Matzner et al. Chem. Review 64, pp 668 et 670 – 1964).

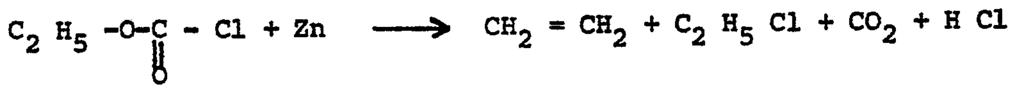

Les halogénoformiates de tétrahalogéno-1,2,2,2 éthyle de départ se préparent facilement, par exemple par réaction d'un trihalogénoacétaldéhyde de formule

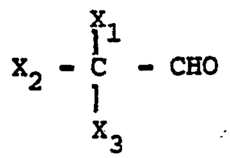

sur un dihalogénure de carbonyle de formule

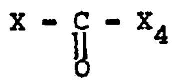

selon le mode opératoire décrit dans le brevet FR 2 482 587.

Selon une variante du procédé on peut remplacer l'halogénoformiate de départ par ses constituants, le trihalogénoacétaldéhyde de formule

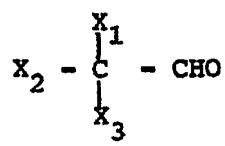

et le dihalogénure de carbonyle de formule

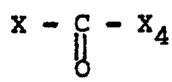

$X_1$, $X_2$, $X_3$ et $X_4$ ayant les significations précédentes.

La deshalogénation s'effectue bien avec les halogénoformiates tel que le chloroformiate de tétrachloro-1,2,2,2 éthyle ou le chloroformiate de chloro-1 tribromo-2,2,2 éthyle.

Comme métal on préfère utiliser le zinc en poudre plutôt que le magnésium en tournures ou en poudre.

Le zinc en poudre est de préférence activé au préalable par exemple selon les méthodes indiquées par Fieser & Fieser dans "Reagents for organic synthesis" New-York (1967) vol. 1 p 1276 ou on peut recourir au zinc cuivré en poudre préparé selon R. Wilkinson dans J. Chem. Soc. (1931) p 3057.

On emploie au moins une quantité stœchiométrique de métal et de préférence un excès de 5 à 50%.

La réaction a lieu dans un solvant ou dans un mélange de solvants, de préférence anhydre. On choisit les solvants parmi les éthers cycliques ou non et les esters, seuls ou en mélange avec les éthers.

Le tétrahydrofuranne, le dioxanne, l'éther diéthylique, le diméthoxyéthane, l'acétate d'éthyle et l'acétate de méthyle conviennent bien.

La température est généralement comprise entre 0°C et +60°C. De préférence on opère entre +5°C et 30°C.

La durée de la réaction est généralement comprise entre 30 minutes et quelques heures. Les solvants sont éliminés par exemple par chauffage ou sous vide. Les sels de zinc peuvent être précipités si nécessaire. L'halogénoformiate de dihalogéno-2,2 vinyle peut être récupéré par distillation.

Le procédé selon l'invention permet, à partir de matières premières d'accès facile, d'obtenir de façon simple et avec de bons rendements de nouveaux halogénoformiates particulièrement utiles en raison de la présence simultanée de la fonction vinylique et de deux atomes d'halogène fixés sur cette fonction.

Grâce à ces caractéristiques ils peuvent servir comme monomères pour former de nouveaux polymères ou comme intermédiaires de synthèse.

L'invention concerne également certaines applications des nouveaux halogénoformiates.

Selon ces applications, les halogénoformiates de dihalogéno-2,2 vinyle sont mis à réagir avec l'ammoniac, une amine primaire ou secondaire, un composé hydroxylé, tel qu'un diol, un alcool ou un phénol pour former des carbamates ou des carbonates dihalogéno-2,2 vinyliques.

Les réactions sont les suivantes:

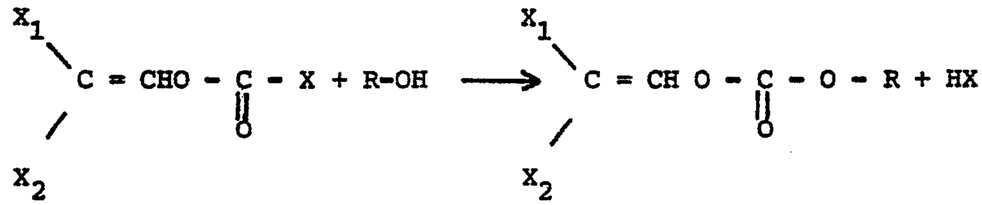

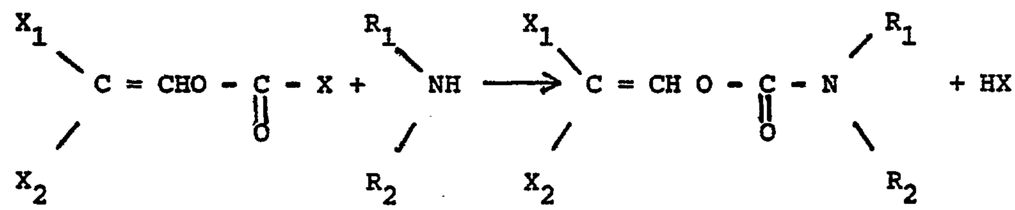

Les conditions de réaction sont classiques (cf. Chemical Review 64, pages 651–657).

Grâce aux nouveaux halogénoformiates selon l'invention les carbamates et carbonates de dihalogéno-2,2 vinyle, en particulier de dichloro-2,2 vinyle, sont obtenus avec un bien meilleur rendement que par le procédé complexe de l'art antérieur (brevet GB 1 221 205). Ces composés sont très utiles pour augmenter l'efficacité des insecticides ou être polymérisés avec d'autres monomères tel que par exemple l'éthylène afin de modifier les propriétés des polymères comme décrit dans le brevet GB 1 221 205.

Les exemples suivants illustrent l'invention sans toutefois la limiter.

Exemple 1

Préparation du chloroformiate de dichloro-2,2 vinyle à partir du chloroformiate de tétrachloro-1,2,2,2 éthyle.

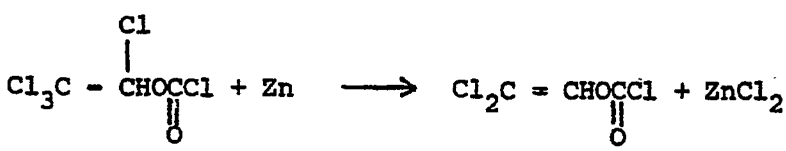

On ajoute par petites portions à l'aide d'un distributeur de poudre et sous agitation, 7,9 g (0,12 mole; 1,09 eq) de zinc en poudre (ALDRICH) à une solution de 27,3 g (0,11 mole) de chloroformiate de tétrachloro-1,2,2,2 éthyle dans 100 ml de THF.

En raison d'une phase initiale d'induction, on n'ajoute une nouvelle portion de poudre de zinc que lorsque la précédente a été consommée.

Après l'addition du zinc, on laisse sous agitation pendant 4 heures puis on évapore sous 0,5 mm Hg et on recueille les volatils dans un piège à −78°C.

Par distillation fractionnée sous pression réduite, on obtient 15 g (rendement 75%) de produit attendu.

Pt Eb.: 82–85°C/120 mm Hg.

I.R. ($CCl_4$) $cm^{-1}$: 3104 (moy.), 1785 (forte), 1131 (très forte)

RMN $^1H$ ($CDCl_3$) $\delta ppm$: 7,50 (s)

RMN $^{13}C$ ($CDCl_3$) $\delta ppm$: 147,3 (d, J = 2,9 Hz, C=O);

134,1 (d, J = 204,9 Hz, =CH–);

116,3 (d, J = 11,8 Hz, $Cl_2C=$)

Exemple 2

Préparation du chloroformiate de dichloro-2,2 vinyle par réaction du phosgène avec le chloral en présence de zinc.

$$CCl_3CHO + COCl_2 + Zn \longrightarrow Cl_2C = CHO\underset{\underset{O}{\|}}{C}Cl + ZnCl_2$$

On introduit 10 ml (0,14 mole; 1,5 eq) de phosgène liquide dans une suspension agitée de 0,1 g de poudre de zinc dans un mélange de 30 ml d'acétate de méthyle (préalablement distillé sur $P_2O_5$) et 15 ml d'éther (séché sur fil de sodium).

On ajoute 15 minutes plus tard 13,8 g (0,0938 mole) de chloral (distillé dans une colonne $CaSO_4$ anhydre selon PERRIN, ARMAREGO, PERRIN dans "Purification of Laboratory Chemicals" 2ème éd. p. 162 Pergamon Press, Londres 1980).

Après consommation de la première portion de poudre de zinc, on ajoute par portions le reste, c'est-à-dire 8 g (0,12 mole), à l'aide d'un distributeur de poudre en téflon. On doit s'assurer que chaque portion de poudre de zinc est consommée avant d'introduire la suivante.

Le milieu réactionnel est maintenu à température ambiante par un bain d'eau froide.

On agite 2 heures puis on élimine l'excès de phosgène par dégazage sous vide. On filtre le résidu liquide sur verre fritté (25–50 µ) et on distille le filtrat orangé sous 1,5 mm Hg à température ambiante dans un piège à –80°C.

Pour extraire les dernières quantités de produit du résidu de distillation, on ajoute 3 ml de nitrobenzène (distillé sur $P_2O_5$) et on poursuit la distillation sous vide.

Après distillation fractionnée du liquide recueilli dans le piège (37–40°C/15 mm Hg), on obtient 8,37 g (rendement 51%) de chloroformiate de dichloro-2,2 vinyle possédant les caractéristiques indiquées à l'exemple 1.

Exemple 3

Préparation du chloroformiate de dibromo-2,2 vinyle à partir du chloroformiate de chloro-1 tribomo-2,2,2 éthyle.

$$CBr_3 - \overset{\overset{Cl}{|}}{C}HO\underset{\underset{O}{\|}}{C}Cl + Zn \longrightarrow Br_2C = CHO\underset{\underset{O}{\|}}{C}Cl + 1/2\ ZnCl_2 + 1/2\ ZnBr_2$$

On ajoute par petites portions à l'aide d'un distributeur de poudre, en 2 heures environ et sous agitation, 1,1 g (0,017 mole; 1,3 eq) de poudre de zinc activé (selon FIESER L.F., FIESER M. "Reagents for Organic Synthesis" Vol. 1 p. 1276 WILEY, NEW-YORK 1967) à une solution de 5,02 g (0,0132 mole) de chloroformiate de chloro-1 tribromo-2,2,2 éthyle dans 10 ml d'acétate d'éthyle (distillé sur $P_2O_5$).

Après 30 minutes d'agitation, on ajoute 10 ml d'un mélange 2/1 pentane/dioxanne (pentane distillé sur sodium-benzophénone, dioxanne distillé sur $LiAlH_4$) pour précipiter les sels de zinc.

Après filtration, on concentre la solution sous vide et on filtre de nouveau. On ajoute ensuite 5 ml de chloro-1 naphtalène et on distille à 35–70°C sous 0,4 mm Hg dans un piège à –78°C.

Le produit obtenu est purifié par distillation fractionnée pour conduire à 1,14 g (rendement 33%) de chloroformiate de dibromo-2,2 vinyle pur.

Pt Eb.: 68–69°C/12 mm Hg
I.R. ($CCl_4$) $cm^{-1}$: 3090 (faible), 1782 (forte)
RMN $^1H$ ($CDCl_3$) δppm: 7,75 (s)
RMN $^{13}C$ ($CDCl_3$) δppm: 147,3 (d, J = 2,7 Hz, C=O);
138,3 (d, J = 204,3 Hz, =CH–);
84,2 (d, J = 12,3 Hz, $Br_2C$=)

Exemple 4

Préparation du chloroformiate de dichloro-2,2 vinyle par réaction du phosgène avec le chloral en présence de magnésium.

On ajoute 5,2 g (0,053 mole; 1,8 eq.) de phosgène à un mélange agité de 4,41 g de chloral (0,030 mole), 0,86 g (0,035 mole; 1,2 eq) de magnésium en poudre (MALLINCKRODT, 40 mesh) et 25 ml d'acétate

d'éthyle. Après 40 minutes d'agitation à une température d'environ 20°C l'analyse RMN$^1$H avec étalon interne montre la formation du chloroformiate de dichloro-2,2 vinyle avec un rendement de 6%.

Exemple 5

Application du chloroformiate de dichloro-2,2 vinyle à la préparation du N-(chloro-3 phényl) carbamate de dichloro-2,2 vinyle.

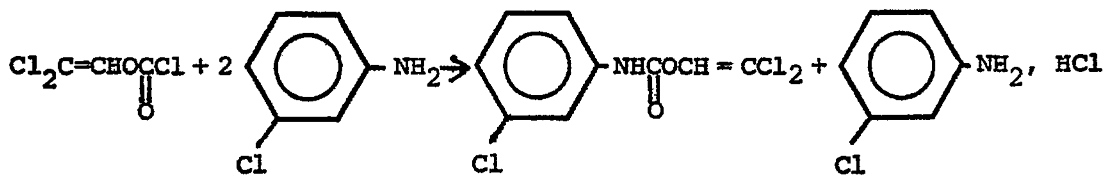

Dans un réacteur de 500 ml, on introduit 17,55 g (0,1 mole) de chloroformiate de dichloro-2,2 vinyle et 60 g d'éther éthylique.

On refroidit à 0°C et ajoute, en 30 min, sous agitation, une solution de 25,5 g (0,2 mole) de chloro-3 aniline dans 50 g d'éther.

On laisse sous agitation 2 heures à la température d'environ 20°C, on filtre, on lave le filtrat à l'eau, on sèche sur $MgSO_4$ et évapore le solvant.

On recueille ainsi 26,5 g (rendement 99%) du carbamate attendu (pureté >98%).

P.F.: 94°C (littérature: 92–93°C exemple 4 du brevet GB 1 221 205)

$$\text{I.R. } (CCl_4)\quad C{=}O : 1775\ cm^{-1}$$

RMN $^1$H ($CDCl_3$) δppm: 7,1 (m, 4H);
7,35 (m, 1H);
7,45 (s, 1H)

Exemple 6

Application du chloroformiate de dichloro-2,2 vinyle à la préparation du carbonate de phényle et de dichloro-2,2 vinyle

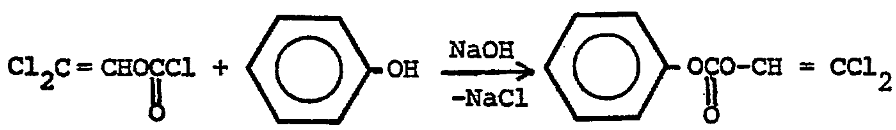


Dans un réacteur de 250 ml, on introduit 10,3 g (0,11 mole) de phénol, 19,3 g (0,11 mole) de chloroformiate de dichloro-2,2 vinyle et 150 g de chloroforme. On refroidit à 0°C, et on coule en 30 min sous agitation 11 g d'une solution aqueuse de soude à 50%.

On laisse ensuite agiter 1 heure à température ambiante, on lave à l'eau et on élimine le solvant par évaporation sous pression réduite.

On recueille ainsi 21,3 g (rendement 83%) du carbonate attendu. (Cf. exemple 7 du brevet GB 1 221 205).

Aspect: solide blanc P.F. 50°C

$$\text{IR } (CCl_4)\quad \begin{cases} C{-}O{-}C : 1250\ cm^{-1} \\ C{=}O : 1785\ cm^{-1} \end{cases}$$

RMN $^1$H ($CDCl_3$) δppm: 7,3 (m, 5H)
7,45 (s, 1H)

Exemple 7

Préparation du chloroformiate de dibromo-2,2 vinyle par réaction du phosgène avec le bromal en présence de zinc.

On a ajouté 10 g (0,15 moles; 0,5 équivalent) de poudre de zinc activé par petites fractions pendant 5 jours à une solution agitée de bromal (80,2 g; 0,29 moles) dans 75 ml d'un mélange 2:1 d'acétate d'éthyle et d'éther en présence de phosgène (38 ml; 0,5 moles). Au bout de 7 jours, un peu de zinc subsistant encore, l'excès de phosgène a été éliminé et le mélange filtré. Les sels de zinc ont été éliminés par un triple lavage avec un mélange d'hexane (50 ml) et de dioxane (30 ml). Les solvants ont été éliminés par distillation simple puis par distillation sous pression réduite. Le produit obtenu a été purifié par distillation fractionnée et présentait les mêmes caractéristiques que le chloroformiate de dibromo-2,2 vinyle obtenu dans l'exemple 3:
I.R. ($CCl_4$) $cm^{-1}$: 3090 (faible), 1780 (forte)
RMN $^1H$ ($CDCl_3$) δppm: 7,75

**Revendications**

1. Halogénoformiates de dihalogéno-2,2 vinyle caractérisés en ce qu'ils sont représentés par la formule:

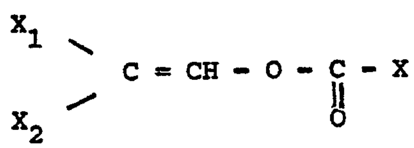

dans laquelle:
X signifie un atome de chlore ou de brome,
$X_1$ et $X_2$ identiques ou différents signifient des atomes de chlore ou de brome.

2. Halogénoformiate selon la revendication 1 caractérisé en ce que $X_1$, $X_2$ et X représentent des atomes de chlore.

3. Halogénoformiate selon la revendication 1 caractérisé en ce que $X_1$ et $X_2$ représentent des atomes de brome et X représente un atome de chlore.

4. Procédé de préparation des halogénoformiates selon l'une quelconque des revendications précédentes caractérisé en ce qu'un halogénoformiate de tétrahalogéno-1,2,2,2 éthyle de formule:

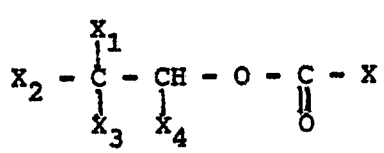

dans laquelle
$X_1$, $X_2$ et X sont tels que définis précédemment,
$X_3$ représente un atome de chlore ou de brome et représente toujours un atome de brome lorsque $X_1$ et/ou $X_2$ représente un atome de brome et $X_4$ représente un atome de chlore ou de brome,
est mis à réagir avec le zinc ou le magnésium dans un milieu solvant.

5. Procédé de préparation selon la revendication précédente caractérisé en ce que le milieu solvant est anhydre.

6. Procédé de préparation selon la revendication 4 ou 5 caractérisé en ce que les composés sont mis à réagir à une température comprise entre 0°C et 60°C.

7. Procédé de préparation selon la revendication précédente caractérisé en ce que la température est comprise entre 5 et 30°C.

8. Procédé selon l'une quelconque des revendications 4 à 7 caractérisé en ce que le métal est utilisé en quantité au moins stoechiométrique.

9. Procédé selon la revendication précédente caractérisé en ce que le métal est utilisé en un excès compris entre 5 et 50%.

10. Procédé selon l'une quelconque des revendications 4 à 9 caractérisé en ce qu'on utilise le zinc en poudre activé ou le zinc en poudre cuivré.

11. Procédé selon l'une quelconque des revendications 4 à 10 caractérisé en ce que le milieu solvant est constitué par un solvant ou un mélange de solvants choisis parmi les éthers cycliques ou non et les esters seuls ou en mélange avec les éthers.

12. Procédé selon la revendication précédente caractérisé en ce que le ou les solvants sont choisis parmi le tétrahydrofuranne, le dioxanne, l'éther diéthylique, le diméthoxyéthane, l'acétate d'éthyle et l'acétate de méthyle.

13. Procédé selon l'une quelconque des revendications 4 à 12 caractérisé en ce que l'on remplace le tétrahalogénoformiate de départ par un trihalogénoacétaldéhyde de formule

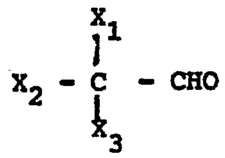

et un dihalogénure de carbonyle de formule

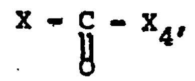

$X_1$, $X_2$, $X_3$ et $X_4$ ayant les significations précédentes.

14. Application des halogénoformiates selon l'une quelconque des revendications 1 à 3 caractérisé en ce qu'ils sont mis à réagir avec un composé hydroxylé pour former un carbonate de dihalogéno-2,2 vinyle.

15. Application des halogénoformiates selon l'une quelconque des revendications 1 à 3 caractérisé en ce qu'ils sont mis à réagir avec l'ammoniac, une amine primaire ou secondaire pour former un carbamate de dihalogéno-2,2 vinyle.

**Patentansprüche**

1. 2,2-Dihalogenvinylhalogenformiate der allgemeinen Formel

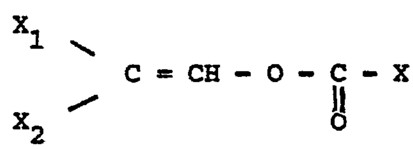

in der bedeuten:
X Chlor oder Brom,
$X_1$ und $X_2$, gleich oder verschieden, je Chlor oder Brom.

2. Halogenformiate nach Anspruch 1, dadurch gekennzeichnet, daß $X_1$, $X_2$ und X Chlor bedeuten.

3. Halogenformiate nach Anspruch 1, dadurch gekennzeichnet, daß $X_1$ und $X_2$ je Brom und X Chlor bedeuten.

4. Verfahren zur Herstellung der Halogenformiate nach einem der vorstehenden Ansprüche, dadurch gekennzeichnet, daß ein 1,2,2,2-Tetrahalogenethylhalogenformiat der Formel

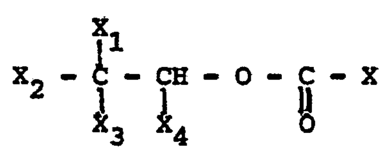

in der
$X_1$, $X_2$ und X wie oben definiert sind,
$X_3$ Chlor oder Brom bedeutet und immer Brom ist, wenn $X_1$ und/oder $X_2$ Brom darstellt und
$X_4$ Chlor oder Brom ist,
mit Zink oder Magnesium in einem Lösungsmittelmedium umgesetzt wird.

5. Herstellungsverfahren nach Anspruch 4, dadurch gekennzeichnet, daß ein wasserfreies Lösungsmittelmedium eingesetzt wird.

6. Herstellungsverfahren nach Anspruch 4 oder 5, dadurch gekennzeichnet, daß die Verbindungen bei einer Temperatur von 0 bis 60°C umgesetzt werden.

7. Herstellungsverfahren nach Anspruch 6, dadurch gekennzeichnet, daß die Verbindungen bei einer Temperatur von 5 bis 30°C umgesetzt werden.

8. Verfahren nach einem der Ansprüche 4 bis 7, dadurch gekennzeichnet, daß das Metall in einer zumindest stöchiometrischen Menge eingesetzt wird.

9. Verfahren nach Anspruch 8, dadurch gekennzeichnet, daß das Metall in einem Überschuß von 5 bis 50% eingesetzt wird.

10. Verfahren nach einem der Ansprüche 4 bis 9, dadurch gekennzeichnet, daß Zink als aktiviertes Pulver oder als kupferhaltiges Pulver eingesetzt wird.

11. Verfahren nach einem der Ansprüche 4 bis 10, dadurch gekennzeichnet, daß ein Lösungsmittelmedium verwendet wird, das aus einem Lösungsmittel oder einem Lösungsmittelgemisch, ausgewählt unter ggfs. cyclischen Ethern und Estern allein oder im Gemisch mit den Ethern, besteht.

12. Verfahren nach Anspruch 11, dadurch gekennzeichnet, daß die Lösungsmittel unter Tetrahydrofuran, Dioxan, Diethylether, Dimethoxyethan, Ethylacetat und Methylacetat ausgewählt werden.

13. Verfahren nach einem der Ansprüche 4 bis 12, dadurch gekennzeichnet, daß das als Ausgangsverbindung verwendete Tetrahalogenformiat durch einen Trihalogenacetaldehyd der Formel

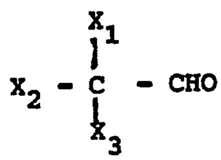

und einem Carbonyldihalogenid der Formel

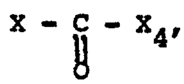

ersetzt wird, wobei $X_1$, $X_2$, $X_3$ und $X_4$ die oben angegebenen Bedeutungen haben.

14. Verwendung der Halogenformiate nach einem der Ansprüche 1 bis 3, dadurch gekennzeichnet, daß sie mit einer Hydroxylgruppen enthaltenden Verbindung zu einem 2,2-Dihalogenvinylcarbonat umgesetzt werden.

15. Verwendung der Halogenformiate nach einem der Ansprüche 1 bis 3, dadurch gekennzeichnet, daß sie mit Ammoniak oder einem primären oder sekundären Amin zu einem 2,2-Dihalogenvinylcarbamat umgesetzt werden.

**Claims**

1. (2,2-Dihalogenovinyl)halogenoformates characterized in that they are represented by the formula:

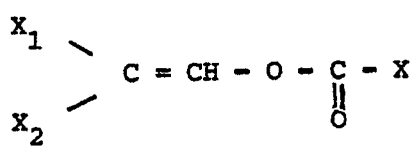

in which:
X represents a chlorine or bromine atom,
$X_1$ and $X_2$ whether identical or different represent chlorine or bromine atoms.

2. Halogenoformate according to Claim 1 characterized in that $X_1$, $X_2$ and X represent chlorine atoms.

3. Halogenoformate according to Claim 1 characterized in that $X_1$ and $X_2$ represent bromine atoms and X represents a chlorine atom.

4. Process for preparing halogenoformates according to any one of the preceding claims characterized in that a (1,2,2,2-tetrahalogenoethyl)-halogenoformate of the formula:

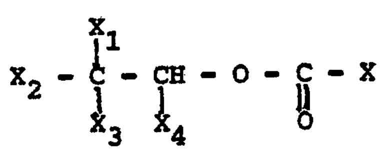

in which
$X_1$, $X_2$ and X are as previously defined,
$X_3$ represents a chlorine or bromine atom and always represents a bromine atom when $X_1$ and/or $X_2$ represents a bromine atom and
$X_4$ represents a chlorine or bromine atom,
is reacted with zinc or magnesium in a solvent medium.

5. Preparative process according to the preceding claim characterized in that the solvent medium is anhydrous.

6. Preparative process according to Claim 4 or 5 characterized in that the compounds are reacted at a temperature in the range 0°C to 60°C.

7. Preparative process according to the preceding claim characterized in that the temperature is in the range 5 to 30°C.

8. Process according to any one of Claims 4 to 7 characterized in that the metal is used in at least the stoichiometric quantity.

9. Process according to the preceding claim characterized in that the metal is used in an excess within the range 5 to 50%.

10. Process according to any one of Claims 4 to 9 characterized in that activated zinc powder or a powdered zinc copper couple is used.

11. Process according to any one of Claims 4 to 10 characterized in that the solvent medium is comprised of a solvent or mixture of solvents selected from ethers which may or may not be cyclic and esters which may or may not be mixed with ethers.

12. Process according to the preceding claim characterized in that the solvent or solvents are selected from tetrahydrofuran, dioxane, diethyl ether, dimethoxyethane, ethyl acetate and methyl acetate.

13. Process according to any one of Claims 4 to 12 characterized in that the initial tetrahalogenoformate is replaced by a trihalogenoacetaldehyde of the formula

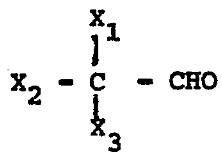

and a carbonyl dihalide of the formula

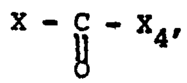

$X_1$, $X_2$, $X_3$ and $X_4$ having the previous meanings.

14. Use of halogenoformates according to any one of Claims 1 to 3 characterized in that they are reacted with a hydroxyl compound to produce a 2,2-dihalogenovinyl carbonate.

15. Use of halogenoformates according to any one of Claims 1 to 3 characterized in that they are reacted with ammonia, a primary or secondary amine to produce a 2,2-dihalogenovinyl carbamate.